# EUROPEAN PATENT SPECIFICATION

(11) **EP 4 307 025 B1**
(45) Date of publication and mention of the grant of the patent: **26.08.2026**
(21) Application number: 22841195.5
(22) Date of filing: 30.06.2022
(51) Int. Cl.: G02B 13/00, G02B 23/24, G02B 13/02, G02B 9/60

(54) **IMAGING DEVICE, OPTICAL LENS, AND ENDOSCOPE**
BILDGEBUNGSVORRICHTUNG, OPTISCHE LINSE UND ENDOSKOP
DISPOSITIF D'IMAGERIE, LENTILLE OPTIQUE ET ENDOSCOPE

(30) Priority: 16.07.2021 CN 202110808035
(43) Date of publication of application: 17.01.2024
(73) Proprietor: ZHEJIANG HEALNOC TECHNOLOGY CO., LTD., Hangzhou Zhejiang 310056 (CN)
(72) Inventor: WU, Pei, Hangzhou, Zhejiang 310051 (CN); YAO, Weizhong, Hangzhou, Zhejiang 310051 (CN); ZHOU, Qiming, Hangzhou, Zhejiang 310051 (CN); LUO, Zhengchun, Hangzhou, Zhejiang 310051 (CN); JIANG, Qingfeng, Hangzhou, Zhejiang 310051 (CN)
(74) Representative: Meyer-Dulheuer MD Legal Patentanwälte PartG mbB
(86) International application number: PCT/CN2022/102731
(87) International publication number: WO 2023/284557

(56) References cited:
- CN-A- 107 526 151
- CN-A- 112 379 508
- CN-A- 113 687 494
- CN-U- 212 965 581
- TW-A- 200 949 288
- TW-A- 202 034 012
- TW-B- I 693 427
- US-A1- 2009 201 592
- US-A1- 2009 201 592
- "Handbook of Optical Systems, Vol 3", 1 January 2007, WILEY-VCH, Weinheim, ISBN: 978-3-52-740379-0, article HERBERT GROSS: "Handbook of Optical Systems, Vol.3 : Aberration Theory and Correction of optical Systems", pages: 377 - 379, XP055169688

## Description

### CROSS-REFERENCE TO RELATED APPLICATIONS

This application claims priority to Chinese patent applications No. 202110808035.8, filed on July 16, 2021, titled "IMAGING DEVICE, OPTICAL LENS, AND ENDOSCOPE".

### TECHNICAL FIELD

The present invention generally relates to a technical field of medical imaging devices, and in particular, to an imaging device, an optical lens, and an endoscope.

### BACKGROUND

Benefit from a rapid development of a smart healthcare filed in recent years, an optical lens has more and more applications in a medical field. Requirements for an optical imaging lens are gradually increased, especially in a field of medical endoscope. In addition, as the camera system gradually develops towards 4K high-definition, a requirement for pixels becomes higher. When an endoscope is applied in the medical field, images of various parts of a body cavity can be obtained by the endoscope inserted into the body cavity, and the image of various parts of a body cavity can be applied to diagnose an observation part.

In the related art, a large-view miniature microscopic objective lens includes a first lens with positive refractive power, a second lens with positive refractive power, a third lens including a lens with negative refractive power and a lens with positive refractive power, a fourth lens with positive refractive power, a fifth lens with negative refractive power, a sixth lens with negative refractive power, and an imaging surface disposed in sequence along an optical axis of the objective lens from an object side to an image side. The lens consists of six elements, the size of the entire lens is too large to complete the miniaturization design, and the pixels are relatively low.

At present, no effective solutions have been proposed for the problems that the entire objective lens has a great size and cannot be designed in miniaturization, and pixels of the objective lens are relatively low in the related art.

An example of a prior art imaging optical system is disclosed in US 2009/0201592 A1. This conventional system discloses a conventional optical length and focal length.

### SUMMARY

The optical lens of the present invention is defined in claim 1, with preferred embodiments specified in the dependent claims. The endoscope of the present invention is defined in claim 11, and the imaging device is defined in claim 12.

According to various embodiments of the present invention, an imaging device, an optical lens, and an endoscope are provided.

The invention provides an optical lens, configured to image an object, including a first lens, a second lens, a third lens, a fourth lens, and a fifth lens; a diaphragm, the first lens, the second lens, the third lens, the fourth lens, the fifth lens, and an imaging surface disposed on the same optical path are provided in sequence from an object side to an image side;
the first lens has positive refractive power, and the first lens includes a first object end surface and a first image end surface;
the second lens has negative refractive power, and the second lens includes a second object end surface and a second image end surface;
the third lens has negative refractive power, and the third lens includes a third object end surface and a third image end surface;
the fourth lens has positive refractive power, and the fourth lens includes a fourth object end surface and a fourth image end surface;
the fifth lens has positive refractive power, and the fifth lens includes a fifth object end surface and a fifth image end surface;
all of the first object end surface, the second object end surface, the fourth object end surface, the fourth image end surface, the fifth object end surface, and the fifth image end surface are convex. All of the first image end surface, the second image end surface, the third object end surface, and the third image end surface are concave; and
the third lens and the fourth lens are combined to form a glued lens set, and the glued lens set satisfies following relational equation: $- 1.1 \leq \frac{f}{{f}_{g 1}} \times tan \left(FOV\right) \leq - 0.2 ,$
*f* represents a focal length of the optical lens, *f*_{*g*1} represents a focal length of the glued lens set, and *FOV* represents a viewing angle of the optical lens.

In some embodiments, the optical lens further includes a filter, and the filter is disposed on an optical path between the fifth lens and the imaging surface.

In some embodiments, the diaphragm is an aperture diaphragm.

In some embodiments, all of the first lens, the second lens, the third lens, the fourth lens, and the fifth lens are spherical lenses. Or, all of the first lens, the second lens, the third lens, the fourth lens, and the fifth lens are aspheric lenses.

In the invention, the optical lens satisfies following relational equation: $TTL / f \leq 1.3 ,$ *TTL* represents a total optical length of the optical lens, and *f* represents a focal length of the optical lens.

In some embodiments, the optical lens satisfies following relational equation: $BFL / TL \leq 0.6 ,$ *BFL* represents an optical back focus of the optical lens, and *TL* represents a lens set length of the optical lens.

In some embodiments, the optical lens satisfies following relational equation: $\frac{\left(R4-R5\right)}{\left(R4+R5\right)} \leq 6.8 ,$ *R4* represents a center radius of curvature of the second image end surface, and *R5* represents a center radius of curvature of the third object end surface.

In some embodiments, materials of the first lens, the second lens, the third lens, the fourth lens, and the fifth lens are glass.

In some embodiments, the optical lens satisfies following conditional equations: ${V}_{d} 1 \leq 82 ;$ ${V}_{d} 2 \leq 65 ;$ ${V}_{d} 4 \leq 71 ,$ *V_{d}1* represents an abbe number of the first lens, *V_{d}2* represents an abbe number of the second lens, and *V_{d}4* represents an abbe number of the fourth lens.

In some embodiments, the optical lens satisfies at least one of following conditional equations: ${N}_{d} 2 \geq 1.45 ;$ ${N}_{d} 3 \leq 1.82 ;$ ${N}_{d} 4 \leq 1.63 ;$ ${N}_{d} 5 \leq 1.87 ,$ *N_{d}2* represents a refractive index of the second lens, *N_{d}3* represents a refractive index of the third lens, *N_{d}4* represents a refractive index of the fourth lens, and *N_{d}5* represents a refractive index of the fifth lens.

In some embodiments, the optical lens satisfies at least one of following conditional equations: $f 1 \leq 21 mm ;$ $f 2 \leq - 31 mm ;$ $f 5 \leq 19 mm ,$ *f1* represents a focal length of the first lens, *f2* represents a focal length of the second lens, and *f5* represents a focal length of the fifth lens.

In a third aspect, the present embodiment provides an endoscope, including a connecting pipe and the optical lens in the second aspect. The connecting pipe is connected to the optical lens and configured to transmit an optical image of the optical lens.

The details of one or more embodiments of the present invention are set forth in the accompanying drawings and the description below. Other features, objects and advantages of the present invention will become apparent from the description, drawings and claims.

### BRIEF DESCRIPTION OF THE DRAWINGS

In order to better describe and explain the embodiments and/or examples of those inventions disclosed herein, one or more drawings may be referred to. The additional details or examples used to describe the drawings should not be considered as limiting the scope of any of the disclosed inventions, the currently described embodiments and/or examples, and the best mode of these inventions currently understood.

In the figures:
FIG. 1 is a schematic view of an optical lens according to one or more embodiments of the present invention.
FIG. 2 is a graph of a modulation transfer function of an optical lens at room temperature in a visible light band according to one or more embodiments.
FIG. 3 is a field curvature view of an optical lens in a visible light band according to one or more embodiments.
FIG. 4 is a distortion view of an optical lens in a visible light band according to one or more embodiments.
FIG. 5 is a first transverse light fan view of an optical lens in a visible light band according to one or more embodiments.
FIG. 6 is a second transverse light fan view of an optical lens in a visible light band according to one or more embodiments.
FIG. 7 is a third transverse light fan view of an optical lens in a visible light band according to one or more embodiments.
FIG. 8 is a fourth transverse light fan view of an optical lens in a visible light band according to one or more embodiments.
FIG. 9 is a fifth transverse light fan view of an optical lens in a visible light band according to one or more embodiments.
FIG. 10 is a first pixel view of light spots of an optical lens in a visible light band according to one or more embodiments.
FIG. 11 is a second pixel view of light spots of an optical lens in a visible light band according to one or more embodiments.
FIG. 12 is a third pixel view of light spots of an optical lens in a visible light band according to one or more embodiments.
FIG. 13 is a fourth pixel view of light spots of an optical lens in a visible light band according to one or more embodiments.
FIG. 14 is a first pixel view of light spots of an optical lens in a visible light band according to one or more embodiments.
FIG. 15 is a graph of modulation transfer function of an optical lens at room temperature in a visible light band according to one or more embodiments.
FIG. 16 is a field curvature view of an optical lens in a visible light band according to one or more embodiments.
FIG. 17 is a distortion view of an optical lens in a visible light band according to one or more embodiments.
FIG. 18 is a schematic view of an imaging device according to one or more embodiments of the present invention.
FIG. 19 is a schematic view of an endoscope according to one or more embodiments of the present invention.

### DETAILED DESCRIPTION OF THE EMBODIMENT

In order to make objects, features and advantages of the present invention more clearly understood, the specific embodiments of the present invention are described in detail below with reference to the accompanying drawings. Obviously, the described embodiments are a part of the embodiments of the present invention, and not all the embodiments. Based on the embodiments in the present invention, all other embodiments obtained by one skilled in the art without creative labor fall within the scope of protection of the present invention. In addition, it should also be understood that although the efforts made in such a development process may be complex and lengthy, some changes in design, manufacture, or production based on the technical contents disclosed in the present invention are merely conventional technical means to one skilled in the art relating to the contents disclosed in the present invention, and should not be construed as inadequate disclosure of the contents disclosed in the present invention.

The reference to "embodiment" in the present invention means that with reference to the particular features, structures or characteristics described in the embodiments may be included in at least one embodiment of the present invention. The phrase "embodiment" appears in various positions in the description does not necessarily refer to the same embodiment, nor is it a separate or embodiment that is mutually exclusive with other embodiments. It can be expressly and implicitly understood by one skilled in the art that the embodiments described in the present invention may be combined with other embodiments in the absence of conflict.

Unless otherwise defined, all technical and scientific terms used herein have the same meaning as a skilled person in the art would understand. In the present invention, when an element is referred to as being "provided on" another element, it may be directly provided on the other element or a further element may be presented between them. When an element is considered to be "disposed on" another element, it may be directly disposed on the other element or a further element may be presented between them. When an element is considered to be "connected to" another element, it may be directly connected to the other element or a further element may be presented between them. The terms "first", "second", "third", etc. involved in the present invention are only configured for distinguishing similar objects, and do not represent a specific order of the objects. The terminology used in the description of the present invention is for the purpose of describing particular embodiments and is not intended to limit the invention. The term "and / or" as used herein includes any and all combinations of one or more of the associated listed items.

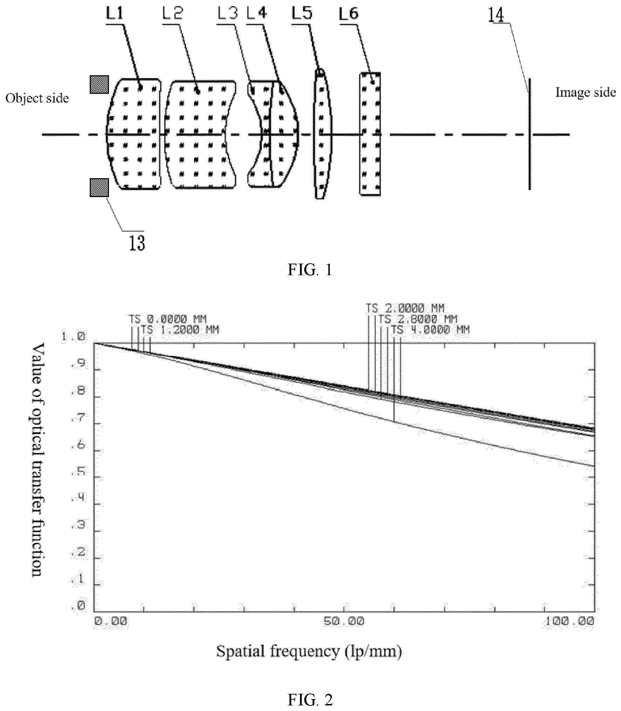

Referring to FIG. 1, the present embodiment provides an optical lens 12, configured to image an object, including a first lens L1, a second lens L2, a third lens L3, a fourth lens L4, and a fifth lens L5. A diaphragm 13, the first lens L1, the second lens L2, the third lens L3, the fourth lens L4, the fifth lens L5, and an imaging surface 14 disposed on the same optical path are provided in sequence from an object side to an image side. The first lens L1 has positive refractive power, and the first lens L1 includes a first object end surface and a first image end surface. The second lens L2 has negative refractive power, and the second lens L2 includes a second object end surface and a second image end surface. The third lens L3 has negative refractive power, and the third lens L3 includes a third object end surface and a third image end surface. The fourth lens L4 has positive refractive power, and the fourth lens L4 includes a fourth object end surface and a fourth image end surface. The fifth lens L5 has positive refractive power, and the fifth lens L5 includes a fifth object end surface and a fifth image end surface. All of the first object end surface, the second object end surface, the fourth object end surface, the fourth image end surface, the fifth object end surface, and the fifth image end surface are convex. All of the first image end surface, the second image end surface, the third object end surface, and the third image end surface are concave. The third lens L3 and the fourth lens L4 are combined to form a glued lens set, and the glued lens set satisfies following relational equation: $- 1.1 \leq \frac{f}{{f}_{g 1}} \times tan \left(FOV\right) \leq - 0.2$ *f* represents a focal length of the optical lens 12, *f*_{*g*1} represents a focal length of the glued lens set, and *FOV* represents a viewing angle of the optical lens 12.

In the present invention, a problem that an entire lens of an imaging device 100 has a great size and cannot be designed in miniaturization can be solved. A specific collocation of surface shape of the optical lens and a reasonable allocation of the focal length of the optical lens can be adopted, thus achieving a more compact structure of the optical lens while satisfying high pixels of the optical lens.

In the present embodiment, the relational equation (1) can limit a structure of the glued lens set and a structure of the entire optical lens 12, specifically the focal length of the entire optical lens 12 and a total optical length of the entire optical lens 12 can be limited, that is, the optical lens can be controlled to design in miniaturization. The structure of the optical lens 12 is as follows. The first lens L1, the second lens L2, the third lens L3, the fourth lens L4, and the fifth lens L5 disposed on the same optical path are provided in sequence from an object side to an image side. The specific collocation of surface shape of each lens of the optical lens can be combined with the reasonable allocation of the focal length of the optical lens satisfying the relational equation (1), thus achieving the more compact structure of the optical lens while satisfying high pixels of the optical lens.

In some embodiments, the optical lens 12 can further include a filter L6, and the filter L6 can be disposed on an optical path between the fifth lens L5 and the imaging surface 14. The filter L6 can protect the first lens L1, the second lens L2, the third lens L3, the fourth lens L4, and fifth lens L5. In addition, the filter L6 has a filtering effect, so that an imaging effect of the optical lens 12 can be further improved. The filter L6 can be usually a planar structure. Lenses of the first lens L1, the second lens L2, the third lens L3, the fourth lens L4, and the fifth lens L5 can be in a variety of structural configurations. For example, the first lens L1, the second lens L2, the third lens L3, the fourth lens L4, and the fifth lens L5 can be spherical lenses. Alternatively, the first lens L1, the second lens L2, the third lens L3, the fourth lens L4, and the fifth lens L5 can be aspheric lenses. It should be noted that the spherical lenses refer to that both an inner side and an outer side of a lens are spherical, or one side of a lens is spherical and the other side of the lens is flat. A surface shape of the aspheric lenses is defined by a polynomial higher-degree equation, and radiuses of points on the surface shape are different from each other. Alternatively, the first lens L1, the second lens L2, the third lens L3, the fourth lens L4, and the fifth lens L5 can be aspheric lenses, so that a thickness of the optical lens 12 can be reduced.

Relational equations or conditional equations that should be satisfied for optical lens 12 are explained in detail below.

In some embodiments, the diaphragm 13 can be an aperture diaphragm. A distance between the aperture diaphragm and the first lens L1 can be less than or equal to 0.4 millimeters. In an embodiment, the distance between the aperture diaphragm and the first lens L1 can be 0.2 millimeters.

In some embodiments, the optical lens 12 can satisfy following relational equation: $TTL / f \leq 1.3$ *TTL* represents a total optical length of the optical lens 12, and *f* represents the focal length of the optical lens 12. In the present embodiment, the optical lens 12 can satisfy the relational equation (1) and the relational equation (2) simultaneously. The total optical length of the optical lens 12 can be controlled by limiting the focal length of the optical lens 12. In this way, the focal length of the optical lens can be reasonably allocated to reduce aberration of the optical lens, and it is conducive to limiting an entire size of the optical lens 12.

In some embodiments, the optical lens 12 can satisfy following relational equation: $BFL / TL \leq 0.6$ *BFL* represents an optical back focus of the optical lens 12, and *TL* represents a lens set length of the optical lens 12. In the present embodiment, the optical lens 12 can satisfy the relational equation (1) and the relational equation (3) simultaneously. The total optical length of the optical lens 12 can be controlled by limiting the optical back focus of the optical lens 12. In this way, the focal length of the optical lens can be reasonably allocated to improve pixels of the optical lens 12, and it is conducive to limiting an entire size of the optical lens 12. In other embodiment, the optical lens 12 can satisfy the relational equation (1), the relational equation (2) and the relational equation (3) simultaneously, which is not limited herein.

In some embodiments, the optical lens 12 can satisfy following relational equation: $\frac{\left(R4-R5\right)}{\left(R4+R5\right)} \leq 6.8$ *R4* represents a center radius of curvature of the second image end surface, and *R5* represents a center radius of curvature of the third object end surface. The center radius of curvature of the second image end surface and the center radius of curvature of the third object end surface can be limited by the relational equation (4), so as to limit a shape of the second lens L2 and a shape of the third lens L3. In this way, both the second lens L2 and the third lens L3 have negative refractive power, which is conducive to reducing apertures and total optical length of the subsequent lenses, thereby realizing miniaturization of the optical lens 12. In an embodiment, the optical lens 12 can satisfy the relational equation (1), the relational equation (2), the relational equation (3) and the relational equation (4) simultaneously. In an embodiment, the optical lens 12 can satisfy the relational equation (1), the relational equation (2) and the relational equation (4) simultaneously. As mentioned above, the more relational equations are satisfied, the better effect the optical lens 12 will achieve, i.e., the more compact the structure of the optical lens 12 is. In addition, image quality of the optical lens 12 can be guaranteed.

In some embodiments, the optical lens 12 can satisfy at least one of following conditional equations: $f 1 \leq 21 mm ; f 2 \leq - 31 mm ; f 5 \leq 19 mm$ *f1* represents a focal length of the first lens L1, *f2* represents a focal length of the second lens L2, and *f5* represents a focal length of the fifth lens L5. In the present embodiment, all the focal length of the first lens L1, the focal length of the second lens L2, and the focal length of the fifth lens L5 can be allocated via the conditional equations (5). An allocation design of the focal length of the optical lens 12 can be further optimized to limit a structure of optical lens 12.

In order to control costs and ensure the optical lens 12 have good processing performance, materials of the first lens L1, the second lens L2, the third lens L3, the fourth lens L4, and the fifth lens L5 can be glass, so that the material of each lens of the optical lens 12 can be required.

In some embodiments, the optical lens 12 can satisfy following conditional equations: ${V}_{d} 1 \leq 82 ; {V}_{d} 2 \leq 65 ; {V}_{d} 4 \leq 71$ *V_{d}1* represents an abbe number of the first lens L1, *V_{d}2* represents an abbe number of the second lens L2, and *V_{d}4* represents an abbe number of the fourth lens L4.

In the present embodiment, the abbe number of the first lens L1, the abbe number of the second lens L2, the abbe number of the fourth lens L4 can be required. The specific requirements can be as follows. The abbe number *V_{d}1* of the first lens L1 can be less than or equal to 82, the abbe number *V_{d}2* of the second lens L2 can be less than or equal to 65, and the abbe number *V_{d}4* of the fourth lens L4 can be less than or equal to 71. For the optical lens 12, these three requirements may be not satisfied simultaneously. For example, one, two, or all of the three requirements may be satisfied. When one of the three requirements is satisfied, the abbe number *V_{d}1* of the first lens L1 can be less than or equal to 82, the abbe number *V_{d}2* of the second lens L2 can be less than or equal to 65, or the abbe number *V_{d}4* of the fourth lens L4 can be less than or equal to 71. When two of the three requirements are satisfied, the abbe number *V_{d}1* of the first lens L1 can be less than or equal to 82 and the abbe number *V_{d}2* of the second lens L2 can be less than or equal to 65, or the abbe number *V_{d}1* of the first lens L1 can be less than or equal to 82 and the abbe number *V_{d}4* of the fourth lens L4 can be less than or equal to 71, or the abbe number *V_{d}2* of the second lens L2 can be less than or equal to 65 and the abbe number *V_{d}4* of the fourth lens L4 can be less than or equal to 71, etc. The other permutations and combinations of the three requirements will not give examples herein. Furthermore, an abbe number of the third lens L3 and an abbe number of the fifth lens L5 are not limited herein. Parameters of the first lens L1, the second lens L2 and the fourth lens L4 can be provided above, so that a distance between adjacent two lenses can be reasonably controlled. In addition, the structure of the optical lens 12 can be more compact, which is conducive to shortening the total optical length of the optical lens 12.

In some embodiments, the optical lens 12 can satisfy at least one of following conditional equations: ${N}_{d} 2 \geq 1.45 ; {N}_{d} 3 \leq 1.82 ; {N}_{d} 4 \leq 1.63 ; {N}_{d} 5 \leq 1.87$ *N_{d}2* represents a refractive index of the second lens L2, *N_{d}3* represents a refractive index of the third lens L3, *N_{d}4* represents a refractive index of the fourth lens L4, and *N_{d}5* represents a refractive index of the fifth lens L5. For the optical lens 12, these four requirements may be not satisfied simultaneously. For example, one, two, three, or all of the three requirements may be satisfied. When one of the four requirements is satisfied, the refractive index *N_{d}2* of the second lens L2 can be greater than or equal to 1.45, and refractive indexes of the other lenses will not be limited. When two of the four requirements are satisfied, the refractive index *N_{d}2* of the second lens L2 can be greater than or equal to 1.45, the refractive index *N_{d}2* of the third lens L3 can be greater than or equal to 1.82, refractive indexes of the other lenses will not be limited. When three of the four requirements are satisfied, the refractive index *N_{d}2* of the second lens L2 can be greater than or equal to 1.45, the refractive index *N_{d}2* of the third lens L3 can be greater than or equal to 1.82, the refractive index *N_{d}5* of the fifth lens L5 can be less than or equal to 1.87, refractive indexes of the other lenses will not be limited. The other permutations and combinations of the four requirements will not give examples herein. Parameters of the second lens L2, the third lens L3 and the fourth lens L4 and the fifth lens L5 can be provided above, so that a distance between adjacent two lenses can be reasonably controlled. In addition, the structure of the optical lens 12 can be more compact, which is conducive to shortening the total optical length of the optical lens 12.

Detailed description of specific embodiments is provided below.

### A first embodiment

The present embodiment provides an optical lens 12, configured to image an object. A diaphragm 13, a first lens L1, a second lens L2, a third lens L3, a fourth lens L4, a fifth lens L5, a filter L6 and an imaging surface 14 disposed on the same optical path are provided in sequence from an object side to an image side. The first lens L1 has positive refractive power, and the first lens L1 includes a first object end surface and a first image end surface. The second lens L2 has negative refractive power, and the second lens L2 includes a second object end surface and a second image end surface. The third lens L3 has negative refractive power, and the third lens L3 includes a third object end surface and a third image end surface. The fourth lens L4 has positive refractive power, and the fourth lens L4 includes a fourth object end surface and a fourth image end surface. The fifth lens L5 has positive refractive power, and the fifth lens L5 includes a fifth object end surface and a fifth image end surface. The filter L6 includes a sixth object end surface and a sixth image end surface. All of the first object end surface, the second object end surface, the fourth object end surface, the fourth image end surface, the fifth object end surface, and the fifth image end surface are convex. All of the first image end surface, the second image end surface, the third object end surface, and the third image end surface are concave. The third lens L3 and the fourth lens L4 are combined to form a glued lens set, and the glued lens set satisfies following relational equation: $- 1.1 \leq \frac{f}{{f}_{g 1}} \times tan \left(FOV\right) \leq - 0.2$ *f* represents a focal length of the optical lens 12, *f*_{*g*1} represents a focal length of the glued lens set, and *FOV* represents a viewing angle of the optical lens 12.

In the present embodiment, relevant parameters of each lens of the optical lens 12 are shown in Table 1, *R* represents a radius of curvature of each lens, *Tc* represents a center thickness of each lens, *N_{d}* represents a d-line refractive index of a material of each lens, and *V_{d}* represents an abbe number of the material of each lens.

**Table 1 (relevant parameters of each lens of the optical lens)**

| Lens name/component name | Radius of curvature *R* (mm) | Center thickness *T_{c}* (mm) | D-line refractive index of a material *N_{d}* | Abbe number of the material *V_{d}* |
|---|---|---|---|---|
| Diaphragm stop | Infinity | 0.2 | | |
| First object end surface | 8.85 | 3.76 | 1.49 | 81.59 |
| First image end surface | 75.81 | 0.34 | | |
| Second object end surface | 11.77 | 4.32 | 1.52 | 60.20 |
| Second image end surface | 6.28 | 2.61 | | |
| Third object end surface | -4.56 | 0.56 | 1.75 | 27.54 |
| Glued surface | 27.03 | 2.01 | 1.59 | 68.34 |
| Fourth image end surface | -6.24 | 1.13 | | |
| Fifth object end surface | 80.73 | 1.27 | 1.84 | 23.79 |
| Fifth image end surface | -17.57 | 1.97 | | |
| Sixth object end surface | Infinity | 1.50 | 1.52 | 64.2 |
| Sixth image end surface | Infinity | 10.65 | | |
| Imaging surface IMA | Infinity | | | |

Based on the relevant parameters of each lens of the optical lens 12, optical specifications of the optical lens 12 in the present embodiment are as follows: an optical length *TTL* of the optical lens 12 can be less than or equal to 30.5 millimeters, a focal length *f* of the optical lens 12 can be 25 millimeters, a viewing angle of the optical lens 12 can be 18 degree, an optical distortion of the optical lens 12 can be 1.0%, an aperture parameter *FNO* of the optical lens 12 can be less than or equal to 4.0, and a diameter of the optical lens 12 at the image end surface can be greater than or equal to 8 millimeters.

It should be noted that a modulation transfer function (MTF) refers to a function that characterizes a relative change between a modulation degree and a lateral phase shift by taking spatial frequency as a variable during imaging an object. The modulation transfer function can be a more accurate, intuitive, and common way to applied to evaluate imaging quality of an imaging system. The higher and smoother a curve of the function is, the better the imaging quality is. Various aberrations (such as spherical aberration, coma, astigmatism, field curvature, axial chromatic aberration, perpendicular chromatic aberration, etc.) can be well corrected.

Referring to FIG. 2, FIG. 2 is a graph of a modulation transfer function of the optical lens 12 at room temperature in a visible light band in the present embodiment. In the FIG. 2, an abscissa of the graph represents the spatial frequency, and an ordinate of the graph represents a value of the modulation transfer function (MTF). A value TS 0.0000MM of the modulation transfer function indicates a center of an imaging area. As shown in FIG. 2, the graph of the modulation transfer function of the optical lens can be smooth and concentrated. An average valve of the MTF in a full viewing angle (a height of a half-image denoted as Y' can be 4.0 millimeters) can be more than 0.6. When the spatial frequency of the optical lens is 100 lp/mm, the valve of the MTF in the full viewing angle can be more than 0.6. It can be seen that an imaging requirement of high-pixels can be achieved.

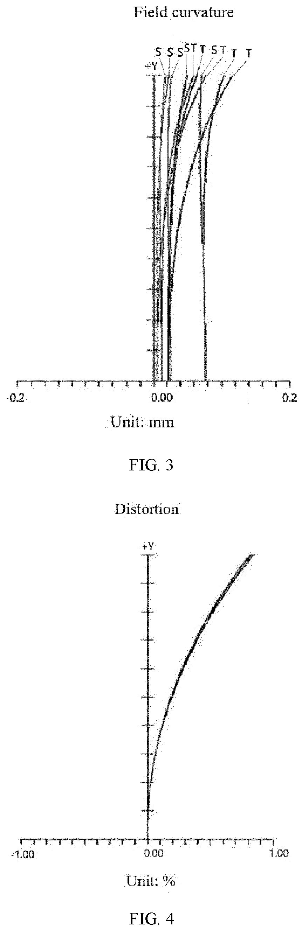

The field curvature can be also known as "an image field curvature". When a field curve is in the lens, an intersection point of an entire beam cannot coincide with an ideal pixel. Although a clear image point can be obtained at each specific point, an entire image plane can be a curved surface. Referring to FIG. 3 and FIG. 4, in the present embodiment, the field curvature can be controlled within ± 0.2 millimeters. In the FIGs, T represents a meridian field curvature and S represents a sagittal field curvature. A field curvature curve can show a distance between a current focal plane of a function of field coordinates and a near-axial focal plane of the function of field coordinates, or a distance between a current image plane of the function of field coordinates and the near-axial focal plane of the function of field coordinates. Data of the meridian field curvature can be measured on the meridian (i.e., YZ plane) along a Z-axis direction, and the data of the meridian field curvature can be a distance between the currently determined focal plane of the function of field coordinates to the near-axis focal plane of the function of field coordinates. Data of the sagittal field curvature can be a distance measured on a plane perpendicular to a meridian plane. A baseline in the graph is on an optical axis of the optical lens 12, top of the curve represents the maximum viewing angle or height of the optical lens 12. The ordinate of the graph has no units, because the curve can be always normalized with a largest radial field of view of the optical lens 12.

Referring to FIG. 4, in the present embodiment, the distortion of the optical lens 12 can be well controlled within 1.0%. The curves in a plurality of wavelengths (i.e., 0.486 millimeters, 0.588 millimeters, 0.656 millimeters, 0.436 millimeters, and 0.900 millimeters) can coincide in FIG. 4. Generally, the distortion of the optical lens can be actually a general term for an inherent perspective distortion of the optical lens. The distortion caused by perspective can be very detrimental to the imaging quality of photos, after all, a purpose of imaging is to reproduce the object, not exaggerate the object. However, since the distortion of the optical lens is an inherent characteristic of the optical lens (a convex lens concentrates light, and a concave lens diverges light), the distortion of the optical lens cannot be eliminated, but can only be improved. In the present embodiment, the distortion of a fixed-focus lens can be only 1.0%, so as to balance the focal length, the viewing angle and a size of a corresponding camera target surface. Deformation of the photos caused by the distortion can be corrected by a post-image processing. Therefore, a detection equipment with a maximum target surface of 1/2 inch can be supported for imaging an object.

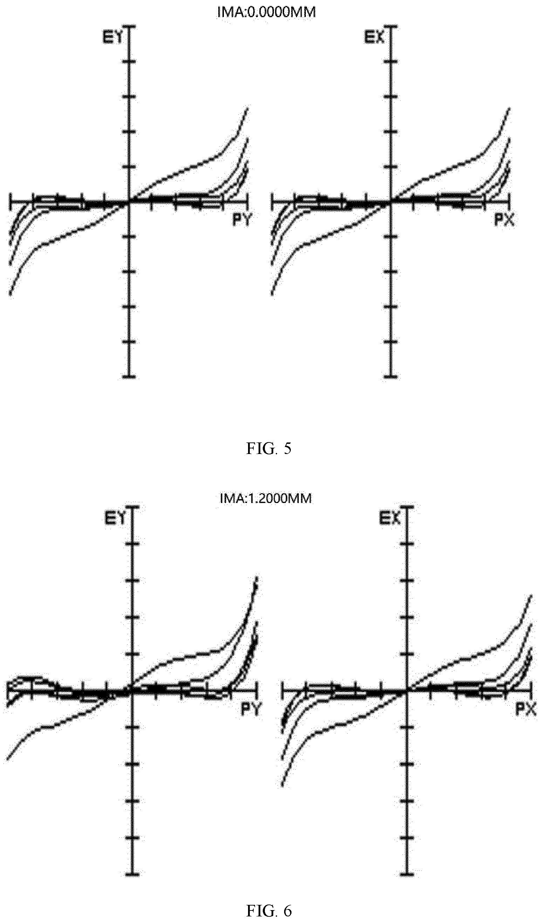

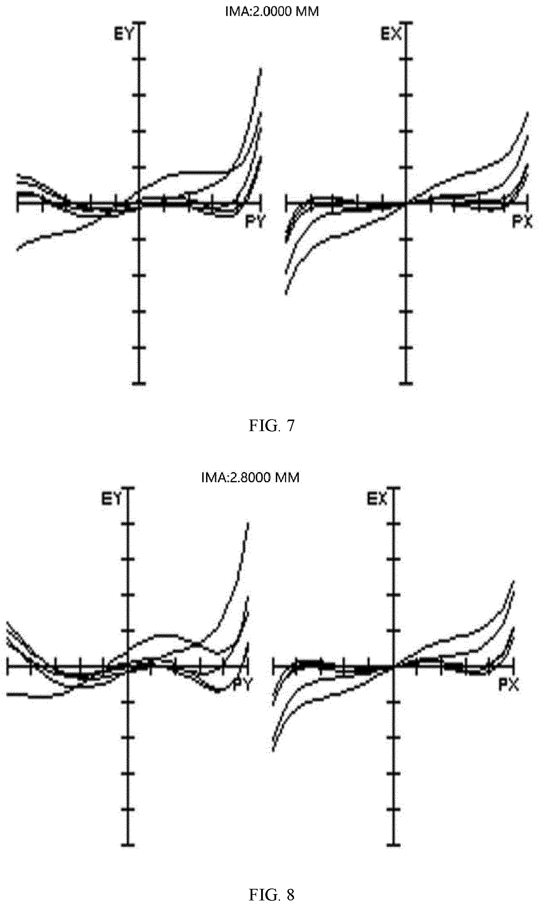

FIG. 5, FIG. 6, FIG. 8 and FIG. 9 show light fan views under each field of view. For example: IMA: 0.000 millimeter represents a main light of zero field of view can intersect the imaging surface 14 (IMA) at a height of 0. EX and EY refer to a difference between a height of a light of the field of view incident to the imaging surface 14 and a height of a main light of the field of view on the imaging surface 14, and the light of the field of view incident to the imaging surface 14 can be a specific pupil in a current light fan. PY represents a pupil coordinate on a meridian light fan, PX represents a pupil coordinate on a sagittal light fan. In addition, the light fan views can be in pairs at each field of view. It can be seen from the above FIGs that curves can be more concentrated, and the spherical aberration and dispersion of the light fan views can be well controlled.

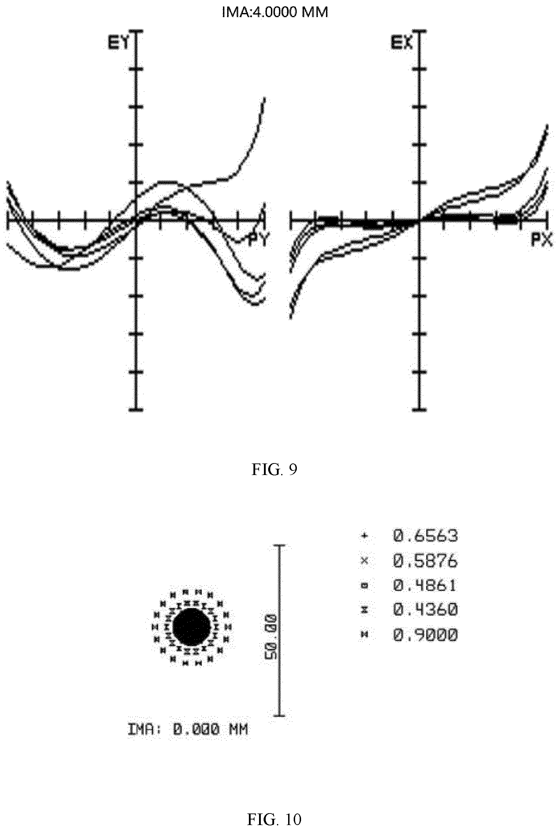

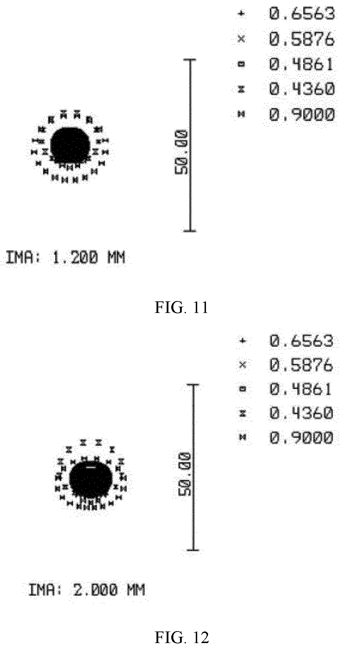

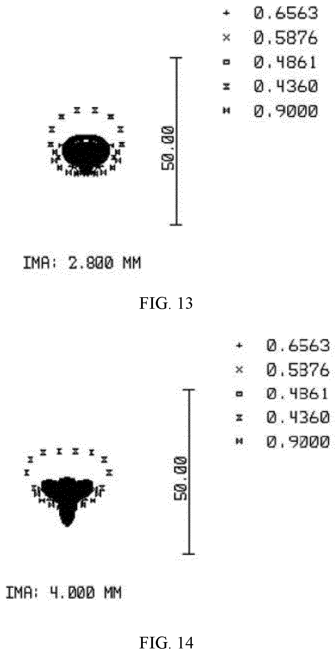

FIG. 10, FIG. 11, FIG. 12, FIG 13 and FIG. 14 show pixel views under each field of view. It can be seen from the above FIGs that a spot radius is relatively small and concentrated, and a corresponding aberration and a corresponding coma can be good.

In the present invention, the specific collocation of surface shape in the optical lens and the reasonable allocation of the focal length in the optical lens can be adopted, thus achieving the more compact structure of the optical lens while satisfying high pixels of the optical lens. A total mechanical length of the optical lens 12 can be less than or equal to 31 millimeters. The number of lenses of the optical lens 12 can be few, processability of the optical lens 12 can be good, and cost of the optical lens 12 can be low. At different temperatures, temperature characteristics of the lenses will compensate with each other, so that the temperature characteristics of the optical lens 12 can be better. Imaging performance of the optical lens 12 will not change significantly at a temperature range of 5°C to 40°C.

### A second embodiment

An optical lens 12 of the second embodiment is the same as that of the optical lens 12 in the first embodiment, and a difference between the second embodiment and the first embodiment is the parameters of each lens of the optical lens 12.

Specifically, in the present embodiment, relevant parameters of each lens of the optical lens 12 are shown in Table 2, *R* represents the radius of curvature of each lens, *T_{c}* represents a center thickness of each lens, *N_{d}* represents a d-line refractive index of a material of each lens, and *V_{d}* represents an abbe number of the material of each lens.

**Table 2 (relevant parameters of each lens of the optical lens)**

| Lens name/component name | Radius of curvature *R* (mm) | Center thickness *T_{c}* (mm) | D-line refractive index of a material *N_{d}* | Abbe number of the material *V_{d}* |
|---|---|---|---|---|
| Diaphragm stop | Infinity | 0.2 | | |
| First object end surface | 7.82 | 2.39 | 1.51 | 63.36 |
| First image end surface | 55.76 | 0.30 | | |
| Second object end surface | 10.64 | 4.16 | 1.53 | 60.20 |
| Second image end surface | 5.95 | 3.00 | | |
| Third object end surface | -4.4 | 0.53 | 1.75 | 27.54 |
| Glued surface | 13.18 | 2.13 | 1.59 | 68.34 |
| Fourth image end surface | -6.18 | 3.83 | | |
| Fifth object end surface | 32.02 | 1.52 | 1.81 | 25.47 |
| Fifth image end surface | -22.80 | 2.48 | | |
| Sixth object end surface | Infinity | 1.50 | 1.52 | 64.20 |
| Sixth image end surface | Infinity | 8.84 | | |
| Imaging surface IMA | Infinity | | | |

Based on the relevant parameters of each lens of the optical lens 12, optical specifications of the optical lens 12 in the present embodiment are as follows: an optical length *TTL* of the optical lens 12 can be less than or equal to 30.5 millimeters, a focal length *f* of the optical lens 12 can be 25 millimeters, a viewing angle of the optical lens 12 can be 18 degree, an optical distortion of the optical lens 12 can be 1.0%, an aperture parameter *FNO* of the optical lens 12 can be less than or equal to 4.0, and a diameter of the optical lens 12 at the image end surface can be greater than or equal to 8 millimeters.

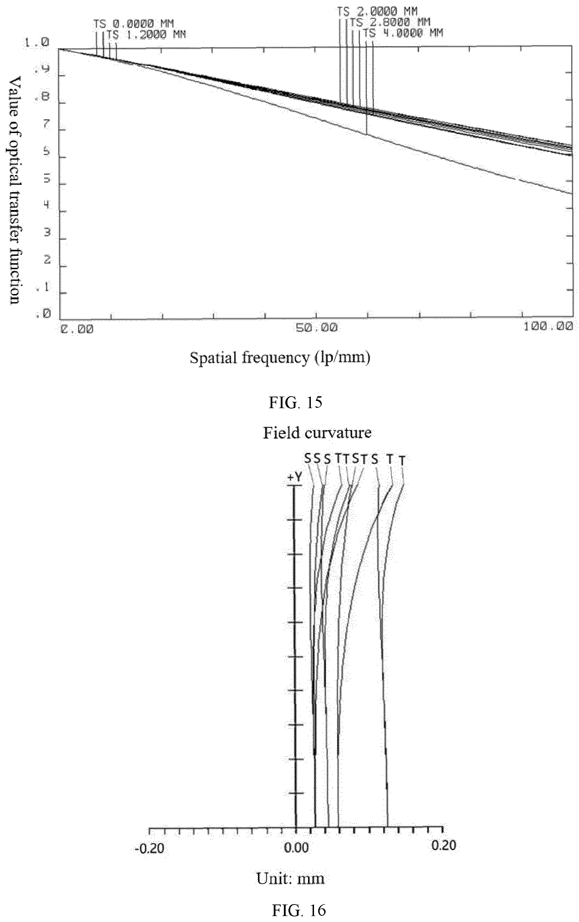

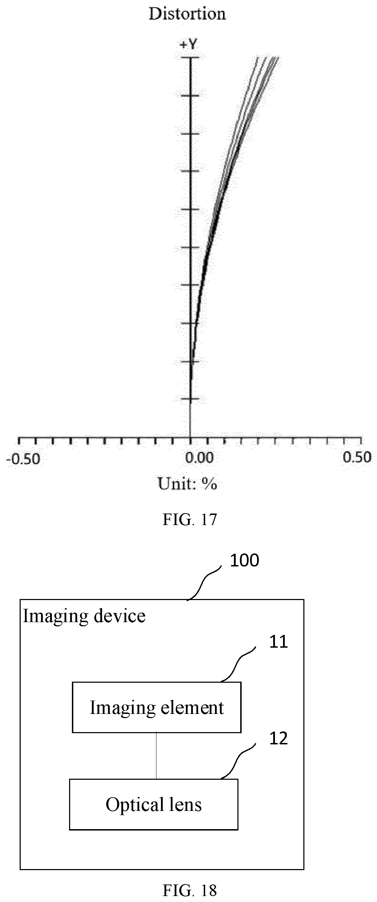

In the present embodiment, referring to FIG. 15, FIG. 16, and FIG. 17, a graph of a modulation transfer function, a field curvature view, and a field curvature view of the optical lens 12 at room temperature in the visible light band are provided, respectively. It can be seen from the FIGs, the optical lens 12 of the present embodiment can also have an effect similar to that in the first embodiment. For example, referring to FIG. 15, the graph of the modulation transfer function of the optical lens can be smooth and concentrated. An average valve of the MTF in a full viewing angle (a height of a half-image denoted as Y' can be 4.0 millimeters) can be more than 0.6. When the spatial frequency of the optical lens is 100 lp/mm, the valve of the MTF in the full viewing angle can be more than 0.6. Referring to FIG. 16, in the present embodiment, the field curvature can be controlled within ± 0.2 millimeters. Referring to FIG. 17, in the present embodiment, the distortion of the optical lens 12 can be well controlled within 1.0%. Therefore, the specific collocation of surface shape in the optical lens and the reasonable allocation of the focal length in the optical lens can be adopted, thus achieving the more compact structure of the optical lens while satisfying high pixels of the optical lens. A total mechanical length of the optical lens 12 can be less than or equal to 31 millimeters. The number of lenses of the optical lens 12 can be few, processability of the optical lens 12 can be good, and cost of the optical lens 12 can be low. When the spatial frequency of the optical lens is 100 lp/mm, the valve of the MTF in the full viewing angle can be more than 0.6. At different temperatures, temperature characteristics of the lenses will compensate with each other, so that the temperature characteristics of the optical lens 12 can be better. Imaging performance of the optical lens 12 will not change significantly at a temperature range of 5°C to 40°C.

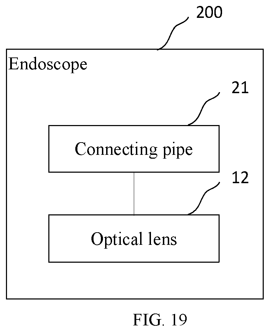

Referring to FIG. 19, the present embodiment provides an endoscope 200, including a connecting pipe 21 and the optical lens 12 of any one of the above embodiments. The connecting pipe 21 is connected to the optical lens 12 and configured to transmit an optical image of the optical lens 12. The connecting pipe 21 can be a fiber insertion pipe or an adjustable delivery pipe. Since the optical lens 12 has characteristics of guaranteeing the imaging quality of photos while miniaturizing the structure of the optical lens 12, good processability, low cost, and a large target surface, so that the endoscope 200 can further have the above characteristics.

Referring to FIG. 18, the present embodiment provides an imaging device 100, including an imaging element 11 and an optical lens 12 of any one of the above embodiments. The imaging element 11 is configured to convert an optical image formed by the optical lens 12 into an electrical signal.

A diaphragm 13, the first lens L1, the second lens L2, the third lens L3, the fourth lens L4, the fifth lens L5, and an imaging surface 14 disposed on the same optical path are provided in sequence from an object side to an image side.

Specifically, the imaging element 11 can be a complementary metal oxide semiconductor (CMOS) image sensor, or a charge coupled device (CCD) image sensor. The imaging device 100 can be a computer or any other electronic device loaded with an optical lens 12. Since the optical lens 12 has characteristics of guaranteeing the imaging quality of photos while miniaturizing the structure of the optical lens 12, good processability, low cost, and a large target surface, so that the imaging device 100 can further have the above characteristics.

Those skilled in the art should understand that the technical features of the above-mentioned embodiments can be combined arbitrarily. In order to make the description concise, not all possible combinations of the technical features are described in the embodiments. However, as long as there is no contradiction in the combination of these technical features, the combinations should be considered as in the scope of the present invention.

The above-described embodiments are merely illustrative of several embodiments of the present invention, and the description thereof is relatively specific and detailed, but is not to be construed as limiting the scope of the invention. It should be noted that a number of variations and modifications may be made by those skilled in the art without departing from the scope of the invention as determined by the appended claims.

## Claims

1. An optical lens (12) for use in an endoscope, configured to image an object on an imaging surface, comprising a diaphragm (13), a first lens (L1), a second lens (L2), a third lens (L3), a fourth lens (L4), and a fifth lens (L5), wherein the diaphragm, the first lens, the second lens, the third lens, the fourth lens, the fifth lens, and the imaging surface disposed on the same optical path are provided in sequence from an object side to an image side;
the first lens has positive refractive power, and the first lens comprises a first object end surface and a first image end surface;
the second lens has negative refractive power, and the second lens comprises a second object end surface and a second image end surface;
the third lens has negative refractive power, and the third lens comprises a third object end surface and a third image end surface;
the fourth lens has positive refractive power, and the fourth lens comprises a fourth object end surface and a fourth image end surface;
the fifth lens has positive refractive power, the fifth lens comprises a fifth object end surface and a fifth image end surface;
all of the first object end surface, the second object end surface, the fourth object end surface, the fourth image end surface, the fifth object end surface, and the fifth image end surface are convex, and all of the first image end surface, the second image end surface, the third object end surface, and the third image end surface are concave;
the third lens and the fourth lens are combined to form a glued lens set, and the glued lens set satisfies following relational equation: $- 1.1 \leq \frac{f}{{f}_{g 1}} \times tan \left(FOV\right) \leq - 0.2 ,$
wherein *f* represents a focal length of the optical lens, *f*_{*g*1} represents a focal length of the glued lens set, and *FOV* represents a viewing angle of the optical lens; and
the optical lens satisfies following relational equation: $TTL / f \leq 1.3 ,$
wherein *TTL* represents a total optical length of the optical lens, and *f* represents a focal length of the optical lens.

2. The optical lens of claim 1, wherein the optical lens further comprises a filter (L6), and the filter is disposed on an optical path between the fifth lens and the imaging surface.

3. The optical lens of claim 1, wherein the diaphragm is an aperture diaphragm.

4. The optical lens of claim 1, wherein all of the first lens, the second lens, the third lens, the fourth lens, and the fifth lens are spherical lenses, or all of the first lens, the second lens, the third lens, the fourth lens, and the fifth lens are aspheric lenses.

5. The optical lens of any one of claims 1 to 4, wherein the optical lens satisfies following relational equation: $BFL / TL \leq 0.6 ,$ wherein *BFL* represents an optical back focus of the optical lens, and *TL* represents a lens set length of the optical lens.

6. The optical lens of any one of claims 1 to 4, wherein the optical lens satisfies following relational equation: $\frac{\left(R4-R5\right)}{\left(R4+R5\right)} \leq 6.8 ,$ wherein *R4* represents a center radius of curvature of the second image end surface, and *R5* represents a center radius of curvature of the third object end surface.

7. The optical lens of any one of claims 1 to 4, wherein materials of the first lens, the second lens, the third lens, the fourth lens, and the fifth lens are glass.

8. The optical lens of claim 7, wherein the optical lens satisfies following conditional equations: ${V}_{d} 1 \leq 82 ;$ ${V}_{d} 2 \leq 65 ;$ ${V}_{d} 4 \leq 71 ,$ wherein *V_{d}1* represents an abbe number of the first lens, *V_{d}2* represents an abbe number of the second lens, and *V_{d}4* represents an abbe number of the fourth lens.

9. The optical lens of claim 7, wherein the optical lens satisfies at least one of following conditional equations: ${N}_{d} 2 \geq 1.45 ;$ ${N}_{d} 3 \leq 1.82 ;$ ${N}_{d} 4 \leq 1.63 ;$ ${N}_{d} 5 \leq 1.87 ,$ wherein *N_{d}2* represents a refractive index of the second lens, *N_{d}3* represents a refractive index of the third lens, *N_{d}4* represents a refractive index of the fourth lens, and *N_{d}5* represents a refractive index of the fifth lens.

10. The optical lens of any one of claims 1 to 4, wherein the optical lens satisfies at least one of following conditional equations: $f 1 \leq 21 mm ;$ $f 2 \leq - 31 mm ;$ $f 5 \leq 19 mm ,$ wherein *f1* represents a focal length of the first lens, *f*2 represents a focal length of the second lens, and *f5* represents a focal length of the fifth lens.

11. An endoscope (200), comprising a connecting pipe (21) and the optical lens of any one of claims 1 to 10, wherein the connecting pipe is connected to the optical lens and configured to transmit an optical image of the optical lens.

12. An imaging device (100), comprising an imaging element and the optical lens of claim 1, wherein the imaging element is configured to convert an optical image formed by the optical lens into an electrical signal, and the optical lens is configured for use in an endoscope.

## Patentansprüche

1. Optische Linse, die so konfiguriert ist, dass sie ein Bild eines Objekts erzeugt, umfassend eine erste Linse, eine zweite Linse, eine dritte Linse, eine vierte Linse und eine fünfte Linse, wobei eine Blende, die erste Linse, die zweite Linse, die dritte Linse, die vierte Linse, die fünfte Linse und eine auf demselben Strahlengang angeordnete Abbildungsfläche nacheinander von einer Objektseite zu einer Bildseite angeordnet sind;
die erste Linse eine positive Brechkraft aufweist und die erste Linse eine erste Objektendfläche und eine erste Bildendfläche umfasst;
die zweite Linse weist eine negative Brechkraft auf, und die zweite Linse umfasst eine zweite Objektendfläche und eine zweite Bildendfläche;
die dritte Linse weist eine negative Brechkraft auf, und die dritte Linse umfasst eine dritte Objektendfläche und eine dritte Bildendfläche;
die vierte Linse weist eine positive Brechkraft auf und umfasst eine vierte Objektendfläche und eine vierte Bildendfläche;
die fünfte Linse weist eine positive Brechkraft auf, und die fünfte Linse umfasst eine fünfte Objektendfläche und eine fünfte Bildendfläche;
sowohl die erste Objektendfläche, die zweite Objektendfläche, die vierte Objektendfläche, die vierte Bildendfläche, die fünfte Objektendfläche als auch die fünfte Bildendfläche sind konvex, und sowohl die erste Bildendfläche, die zweite Bildendfläche, die dritte Objektendfläche als auch die dritte Bildendfläche sind konkav;
die dritte Linse und die vierte Linse sind zu einem geklebten Linsensatz zusammengefügt, und der geklebte Linsensatz erfüllt die folgende Relation: $- 1.1 \leq \frac{f}{{f}_{g 1}} \times tan \left(FOV\right) \leq - 0.2 ,$
wobei *f* eine Brennweite der optischen Linse darstellt, *f*_{*g*1} eine Brennweite des geklebten Linsensatzes darstellt und *FOV* einen Sichtwinkel der optischen Linse im darstellt; und
die optische Linse die folgende Gleichung erfüllt: $TLL / f \leq 1.3 ,$
wobei *TTL* die optische Gesamtlänge der optischen Linse und *f* die Brennweite der optischen Linse bezeichnet.

2. Optische Linse nach Anspruch 1, wobei die optische Linse ferner ein Filter (L6) umfasst und das Filter auf einem Strahlengang zwischen der fünften Linse und der Abbildungsfläche angeordnet ist.

3. Optische Linse nach Anspruch 1, wobei die Blende eine Aperturblende ist.

4. Optische Linse nach Anspruch 1, wobei die erste Linse, die zweite Linse, die dritte Linse, die vierte Linse und die fünfte Linse alle sphärische Linsen sind oder die erste Linse, die zweite Linse, die dritte Linse, die vierte Linse und die fünfte Linse alle asphärische Linsen sind.

5. Optische Linse nach einem der Ansprüche 1 bis 4, wobei die optische Linse die folgende Gleichung erfüllt: $BFL / TL \leq 0.6 ,$ wobei *BFL* den optischen Hinterfokus der optischen Linse und *TL* die Linsensatzlänge der optischen Linse bezeichnet.

6. Optische Linse nach einem der Ansprüche 1 bis 4, wobei die optische Linse die folgende Gleichung erfüllt: $\frac{\left(R4-R5\right)}{\left(R4+R5\right)} \leq 6.8 ,$ wobei *R4* den mittleren Krümmungsradius der zweiten Bildendfläche und *R5* den mittleren Krümmungsradius der dritten Objektendfläche bezeichnet.

7. Optische Linse nach einem der Ansprüche 1 bis 4, wobei die Materialien der ersten Linse, der zweiten Linse, der dritten Linse, der vierten Linse und der fünften Linse aus Glas bestehen.

8. Optische Linse nach Anspruch 7, wobei die optische Linse die folgenden bedingten Gleichungen erfüllt: ${V}_{d} 1 \leq 82 ;$ ${V}_{d} 2 \leq 65 ;$ ${V}_{d} 4 \leq 71 ,$ wobei *V_{d} 1* eine Abbe-Zahl der ersten Linse, *V_{d}* 2 eine Abbe-Zahl der zweiten Linse und *V_{d} 4* eine Abbe-Zahl der vierten Linse darstellt.

9. Optische Linse nach Anspruch 7, wobei die optische Linse mindestens eine der folgenden bedingten Gleichungen erfüllt: ${N}_{d} 2 \geq 1 , 45 ;$ ${N}_{d} 3 \geq 1 , 82 ;$ ${N}_{d} 4 \geq 1 , 63 ;$ ${N}_{d} 5 \geq 1 , 87 ,$ wobei *N_{d} 2* den Brechungsindex der zweiten Linse, *N_{d} 3* den Brechungsindex der dritten Linse, *N_{d} 4* den Brechungsindex der vierten Linse und *N_{d} 5* den Brechungsindex der fünften Linse darstellt.

10. Optische Linse nach einem der Ansprüche 1 bis 4, wobei die optische Linse mindestens eine der folgenden bedingten Gleichungen erfüllt: $f 1 \leq 21 mm ;$ $f 2 \leq - 31 mm ;$ $f 5 \leq 19 mm ,$ wobei *f1* die Brennweite der ersten Linse, *f2* die Brennweite der zweiten Linse und *f5* die Brennweite der fünften Linse bezeichnet.

11. Endoskop (200), umfassend ein Verbindungsrohr (21) und die optische Linse gemäß einem der Ansprüche 1 bis 10, wobei das Verbindungsrohr mit der optischen Linse verbunden und so ausgebildet ist, dass es ein optisches Bild der optischen Linse überträgt.

12. Bildgebungsvorrichtung (100), umfassend ein Bildgebungselement und die optische Linse nach Anspruch 1, wobei das Bildgebungselement so ausgelegt ist, dass es ein von der optischen Linse erzeugtes optisches Bild in ein elektrisches Signal umwandelt, und die optische Linse für die Verwendung in einem Endoskop ausgelegt ist.

## Revendications

1. Lentille optique, configurée pour image d'un objet, comprenant une première lentille, une deuxième lentille, une troisième lentille, une quatrième lentille et une cinquième lentille, dans laquelle un diaphragme, la première lentille, la deuxième lentille, la troisième lentille, la quatrième lentille, la cinquième lentille et une surface de formation d'image disposée sur le même trajet optique sont agencés en séquence depuis le côté objet vers le côté image;
la première lentille présente une puissance de réfraction positive, et la première lentille comprend une première surface d'extrémité objet et une première surface d'extrémité image;
la deuxième lentille présente un pouvoir de réfraction négatif, et comprend une deuxième surface d'objet et une deuxième surface d'image;
la troisième lentille présente une puissance de réfraction négative, et la troisième lentille comprend une troisième surface d'extrémité objet et une troisième surface d'extrémité image;
la quatrième lentille a une puissance de réfraction positive, et la quatrième lentille comprend une quatrième surface d'objet et une quatrième surface d'image;
la cinquième lentille présente une puissance de réfraction positive, et la cinquième lentille comprend une cinquième surface d'objet et une cinquième surface d'image;
la première surface d'objet, la deuxième surface d'objet, la quatrième surface d'objet, la quatrième surface d'image, la cinquième surface d'objet et la cinquième surface d'image sont toutes convexes, et la première surface d'image, la deuxième surface d'image, la troisième surface d'objet et la troisième surface d'image sont toutes concaves;
la troisième lentille et la quatrième lentille sont assemblées pour former un ensemble de lentilles collées, et cet ensemble de lentilles collées satisfait à l'équation relationnelle suivante: $- 1.1 \leq \frac{f}{{f}_{g 1}} \times tan \left(FOV\right) \leq - 0.2 ,$
où *f* représente la distance focale de la lentille optique, *f*_{*g*1} représente la distance focale de l'ensemble de lentilles collées, et *FOV* représente l'angle de champ de la lentille optique; et
la lentille optique satisfait à l'équation relationnelle suivante: $TLL / f \leq 1.3 ,$
où *TTL* représente la longueur optique totale de la lentille optique, et *f* représente la distance focale de la lentille optique.

2. Lentille optique selon la revendication 1, dans laquelle la lentille optique comprend en outre un filtre (L6), et le filtre est disposé sur un trajet optique entre la cinquième lentille et la surface de formation d'image.

3. Lentille optique selon la revendication 1, dans laquelle le diaphragme est un diaphragme d'ouverture.

4. Lentille optique selon la revendication 1, dans laquelle la première lentille, la deuxième lentille, la troisième lentille, la quatrième lentille et la cinquième lentille sont toutes des lentilles sphériques, ou la première lentille, la deuxième lentille, la troisième lentille, la quatrième lentille et la cinquième lentille sont toutes des lentilles asphériques.

5. Lentille optique selon l'une quelconque des revendications 1 à 4, dans laquelle la lentille optique satisfait à l'équation relationnelle suivante: $BFL / TL \leq 0.6 ,$ où *BFL* représente la distance focale arrière de la lentille optique, et *TL* représente la longueur de l'ensemble de lentilles de la lentille optique.

6. Lentille optique selon l'une quelconque des revendications 1 à 4, dans laquelle la lentille optique satisfait à l'équation relationnelle suivante: $\frac{\left(R4-R5\right)}{\left(R4+R5\right)} \leq 6.8 ,$ où *R4* représente le rayon de courbure central de la deuxième surface d'extrémité de l'image, et *R5* représente le rayon de courbure central de la troisième surface d'extrémité de l'objet.

7. Lentille optique selon l'une quelconque des revendications 1 à 4, dans laquelle les matériaux de la première lentille, de la deuxième lentille, de la troisième lentille, de la quatrième lentille et de la cinquième lentille sont du verre.

8. Lentille optique selon la revendication 7, dans laquelle la lentille optique satisfait aux équations conditionnelles suivantes: ${V}_{d} 1 \leq 82 ;$ ${V}_{d} 2 \leq 65 ;$ ${V}_{d} 4 \leq 71 ,$ où *V_{d} 1* représente un indice d'Abbe de la première lentille, *V_{d} 2* représente un indice d'Abbe de la deuxième lentille, et *V_{d} 4* représente un indice d'Abbe de la quatrième lentille.

9. Lentille optique selon la revendication 7, dans laquelle la lentille optique satisfait au moins l'une des équations conditionnelles suivantes: ${N}_{d} 2 \geq 1 , 45 ;$ ${N}_{d} 3 \leq 1 , 82 ;$ ${N}_{d} 4 \leq 1 , 63 ;$ ${N}_{d} 5 \leq 1 , 87 ,$ où *N_{d} 2* représente l'indice de réfraction de la deuxième lentille, *N_{d} 3* représente l'indice de réfraction de la troisième lentille, *N_{d} 4* représente l'indice de réfraction de la quatrième lentille et *N_{d} 5* représente l'indice de réfraction de la cinquième lentille.

10. Lentille optique selon l'une quelconque des revendications 1 à 4, dans laquelle la lentille optique satisfait au moins l'une des équations conditionnelles suivantes: $f 1 \leq 21 mm ;$ $f 2 \leq - 31 mm ;$ $f 5 \leq 19 mm ,$ où *f1* représente la distance focale de la première lentille, *f2* représente la distance focale de la deuxième lentille et *f5* représente la distance focale de la cinquième lentille.

11. Endoscope (200) comprenant un tube de raccordement (21) et la lentille optique selon l'une quelconque des revendications 1 à 10, dans lequel le tube de raccordement est relié à la lentille optique et est configuré pour transmettre une image optique de la lentille optique.

12. Dispositif d'imagerie (100), comprenant un élément d'imagerie et la lentille optique selon la revendication 1, dans lequel l'élément d'imagerie est configuré pour convertir une image optique formée par la lentille optique en un signal électrique, et la lentille optique est configurée pour être utilisée dans un endoscope.
